# EUROPÄISCHE PATENTSCHRIFT

(11) **EP 0 380 778 B1**
(45) Veröffentlichungstag und Bekanntmachung des Hinweises auf die Patenterteilung: **02.03.1994**
(21) Anmeldenummer: 89122181.4
(22) Anmeldetag: 01.12.1989
(51) Int. Cl.: C08G 16/02, C07C 309/28

(54) **Verfahren zur Herstellung von Kondensaten aus Arylsulfonsäuren und Formaldehyd und ihre Verwendung**
Process for preparing condensates of arylsulphonic acids and formaldehyde, and their use
Procédé de préparation de condensats d'acides arylsulphoniques et de formaldéhydes, leur utilisation

(30) Priorität: 14.12.1988 DE 3841987
(43) Veröffentlichungstag der Anmeldung: 08.08.1990
(73) Patentinhaber: BASF Aktiengesellschaft, 67063 Ludwigshafen (DE)
(72) Erfinder: Brückmann, Ralf, Dr., D-6701 Goennheim (DE); Dix, Johannes Peter, Dr., D-6719 Weisenheim (DE); Fikentscher, Rolf, Dr., D-6700 Ludwigshafen (DE); Herrmann, Manfred, Dr., D-6700 Ludwigshafen (DE); Zimmermann, Norbert, D-6701 Waldsee (DE)

(56) Entgegenhaltungen:
- JP-A-58 179 218
- DATABASE WPIL, Accession Nr. 85-258930 [42], Derwent Publications Ltd, London, GB; & JP-A-60 171 257

## Beschreibung

Kondensate aus Arylsulfonsäuren, insbesondere α- und β-Naphthalinsulfonsäuren, sind seit langem bekannt. Aus der DE-PS 1 137 005 ist ein Verfahren zur Herstellung von Kondensationsprodukten aus α- und β-Naphthalinsulfonsäure sowie Methylnaphthalinsulfonsäuren und den entsprechenden Naphthalindisulfonsäuren mit Aldehyden bekannt. Die Kondensation wird bei Temperaturen zwischen 100 und 200°C und erhöhtem Druck durchgeführt. Die so erhältlichen Kondensationsprodukte werden als Hilfsmittel in der Gerberei, als Dispergiermittel für Farbstoffe sowie bei der Papierherstellung zur Vermeidung von Harzschwierigkeiten eingesetzt.

Aus der US-PS 3 277 162 ist die Herstellung von wasserlöslichen Kondensationsprodukten aus Naphthalinsulfonsäuren und Formaldehyd bekannt. Gemäß der Lehre dieser Patentschrift wird zunächst Naphthalin mit konzentrierter Schwefelsäure, z.B. 96 %iger Schwefelsäure, bei Temperaturen zwischen 70 und 175°C sulfoniert. Dabei entstehen - je nach Temperatur bei der Reaktion - 1- und 2-Naphthalinsulfonsäuren. Das Verhältnis der Isomeren in der Mischung der beiden Naphthalinsulfonsäuren ist für die Herstellung von Kondensationsprodukten mit Formaldehyd nicht kritisch. Die so erhältlichen Kondensationsprodukte werden als oberflächenaktive Mittel oder als Retarder beim Färben von Polyacrylnitrilfasern verwendet.

Aus der JP-OS 83-179218 ist ein Verfahren zur Herstellung von Kondensationsprodukten aus Naphthalinsulfonsäuren und Formaldehyd bekannt, bei dem man Naphthalin mit rauchender Schwefelsäure sulfoniert und die Sulfonierungsprodukte anschließend mit Formaldehyd unter Inertgasdruck mit Formaldehyd kondensiert. Vorzugsweise setzt man bei diesem Verfahren reines Naphthalin ein, kann jedoch auch Alkylnaphthaline verwenden. Die so hergestellten Produkte werden als Dispergiermittel für Zement verwendet.

Aus der DE-PS 27 45 449 sind stabile feindisperse wäßrige Zübereitungen von Dispersionsfarbstoffen und von in Wasser schwer bis unlöslichen optischen Aufhellern bekannt, die als Dispergiermittel sulfonierte Anlagerungsprodukte von Alkylenoxiden an spezielle Phenole enthalten. Die darin beschriebenen feindispersen Zübereitungen von Dispersionsfarbstoffen erlauben einwandfreie egale Wickelkörperfärbungen ohne Abfiltration von Farbstoff während des Färbeprozesses. Wie aus dieser Literaturstelle bekannt ist, eignen sich für Wickelkörperfärbungen außer den darin beschriebenen Dispergiermitteln lediglich noch Ligninsulfonate.

Ligninsulfonate sind jedoch nicht bei allen Farbstoffen wirksam und haben die Eigenschaft, empfindliche Azofarbstoffe beim Färbevorgang zu reduzieren, so daß man beim Färben stark verminderte Farbausbeuten erhält. Als besonders ungünstig in bezug auf die Wickelkörperfärbung gelten solche Farbstoffzubereitungen, die Kondensationsprodukte aus Naphthalinsulfonsäure und Formaldehyd als Dispergiermittel enthalten.

Der vorliegenden Erfindung liegt die Aufgabe zugrunde, ein Verfahren zur Herstellung von Kondensaten aus Arylsulfonsäuren und Formaldeyhd zur Verfügung zu stellen, bei dem man Kondensationprodukte erhält, die gegenüber den bisher beschriebenen Kondensationsprodukten dieser Art die Herstellung stabiler Farbstoffzubereitungen gestattet, die einwandfreie egale Wickelkörperfärbungen ohne Abfiltration von Farbstoff während des Färbeprozesses ergeben.

Die Aufgabe wird erfindungsgemäß gelöst mit einem Verfahren zur Herstellung von Kondensaten aus Arylsulfonsäuren und Formaldehyd durch Sulfonieren von Arylverbindungen und Kondensieren der Arylsulfonsäuren mit Formaldehyd in wäßrigem Medium, wenn man
(a) bei der Sulfonierung als Arylverbindungen die bei 100 bis 120°C und 1013 mbar anfallende Fraktion einsetzt, die bei der fraktionierten Destillation der Produkte der thermischen Spaltung eines naphthenischen Rückstandsöls erhalten wird,
(b) die Sulfonierung mit Oleum bei Temperaturen von 120 bis 160°C durchführt und
(c) pro Gewichtsteil der Arylverbindungen gemäß (a) 0,7 bis 1,2 Gewichtsteile, vorzugsweise 0,8 bis 0,9 Gewichtsteile Oleum, bezogen auf Oleum mit einem SO₃-Gehalt von 24 Gew.%, anwendet.

Die so herstellbaren Kondensationsprodukte sind wirksame Dispergiermittel und erlauben die Herstellung von wäßrigen Farbstoffzubereitungen, die über einen längeren Zeitraum lagerstabil sind und die auch für die Herstellung egaler Wickelkörperfärbungen ohne eine Abfiltration von Farbstoff während des Färbevorgangs verwendet werden können.

Bei der Sulfonierung werden Arylverbindungen eingesetzt, die durch thermische Spaltung eines naphthenischen Rückstandsöl und Fraktionieren der Spaltprodukte erhältlich sind. Die naphthenischen Rückstandsöle fallen beispielsweise beim Cracken von Leichtbenzin an. Sie werden z.B. in der DE-OS 29 47 005 als hochsiedende aromatische Kohlenwasserstofföle bezeichnet.

Das naphthenische Rückstandsöl wird vorzugsweise bei Temperaturen von 1400 bis 1700°C thermisch gespalten. Die Spaltprodukte werden einer fraktionierten Destillation zugeführt. Die bei Normaldruck (1013 mbar) von 100 bis 120°C siedende Fraktion wird gesammelt und sulfoniert. Diese Fraktion wird bei dem bekannten Acetylen-Oil-Quench-Prozeß üblicherweise als Nebenprodukt erhalten, vgl. Ullmanns Encyclopedia of Industrial Chemistry, VCH Verlagsgesellschaft mbH, Weinheim, 1985, Volume 71, Seiten 107-112.

Diese Aromatenfraktion besteht aus einer Mischung vieler aromatischer Substanzen, deren Struktur und Menge praktisch nicht im einzelnen ermittelt werden kann. Folgende Arylverbindungen sind die hauptsächlichsten Vertreter dieser Aromatenfraktion:

| | Gew.% in der Aromatenfraktion |
|---|---|
| Naphthalin | 30-55 |
| 2-Methylnaphthalin | 5-15 |
| 1-Methylnaphthalin | 4-10 |
| Inden | 3-10 |
| Diphenyl | 1- 5 |
| Methylinden | 1- 5 |
| Acenaphthen | 1- 4 |

Die Aromatenfraktion enthält außerdem an identifizierten Bestandteilen in Mengen von 0,1 bis etwa 2 Gew.% folgende Arylverbindungen: Fluoren, Indan, Methylstyrol, Phenanthren, Methylindan, Dimethylnaphthalin, Ethylnaphthalin, Xylole, Tetralin, Styrol, Methylethylbenzol, Anthracen, Fluoranthren, Pyren, Acetnaphthylen und Toluol.

Bei der Sulfonierung wird vorzugsweise eine Aromatenfraktion eingesetzt, die 40 bis 45 Gew.% Naphthalin enthält. Die oben beschriebene Fraktion von Arylverbindungen, die eine Mischung vieler aromtischer Substanzen darstellt, wird mit Oleum bei Temperaturen von 120 bis 160, vorzugsweise 135 bis 145°C sulfoniert. Bei höheren Temperaturen werden kürzere Reaktionszeiten benötigt als bei niedrigeren Temperaturen. Beispielsweise ist die Sulfonierung bei einer Temperatur von 145°C innerhalb eines Zeitraumes von 1,6 bis 2,6 Stunden beendet, während man bei einer Temperatur von 140°C dafür 2,25 bis 4 Stunden und bei 135°C 3,25 bis 6 Stunden benötigt. Ein höherer molarer Überschuß an Oleum, bezogen auf das in der Aromatenfraktion enthaltene Naphthalin, führt ebenso zu einer Verkürzung der Reaktionszeit. So benötigt man beispielsweise bei einem 10 %igen molaren Überschuß an Oleum bei einer konstanten Temperatur von 140°C Reaktionszeiten von 2,45 bis 4 Stunden und bei einem 20 %igen Überschuß an Oleum bei derselben Temperatur eine Reaktionsdauer von 1,4 bis 3 Stunden.

Die Bedingungen bei der Sulfonierung der gemäß (a) erhaltenen Aromatenfraktion sind derart, daß das darin enthaltene Naphthalin zu α- und zu β-Naphthalinsulfonsäuren umgesetzt wird, wobei das Verhältnis von α- zu β-Naphthalinsulfonsäure 10:1 bis 1:2 beträgt. Pro Gewichtsteil der Arylverbindungen gemäß (a) setzt man bei der Sulfonierung 0,7 bis 1,2 Gewichtsteile Oleum ein, bezogen auf rauchende Schwefelsäure mit einem SO₃-Gehalt von 24 Gew.%. Es kann selbstverständlich auch Schwefelsäure mit anderen SO₃-Gehalten eingesetzt werden, z.B. Oleum mit einem Gehalt von 10 bis 65 Gew.% SO₃. Bei geringeren SO₃-Gehalten im Oleum benötigt man für die Sulfonierung entsprechend dem SO₃-Gehalt eine höhere Menge an Oleum als bei Einsatz von 24 %igem Oleum, während man bei höher konzentriertem Oleum entsprechend weniger einsetzen muß. Die Menge an Oleum richtet sich nach dem SO₃-Gehalt in der rauchenden Schwefelsäure. Die Einhaltung der Temperatur bei der Sulfonierung und die Menge an Oleum, bezogen auf die Arylverbindungen gemäß (a), führen zu Sulfonierungsprodukten, die α- und β-Naphthalinsulfonsäure im Verhältnis von 20:1 bis 1:3, vorzugsweise 10:1 bis 1:2 enthalten.

Die sulfonierten Arylverbindungen werden anschließend in üblicher Weise mit Formaldehyd kondensiert. Hierzu kann man direkt vom Sulfonierungsgemisch ausgehen, es mit Wasser verdünnen und durch Zugabe von Formaldehyd in dem Temperaturbereich von 90 bis 105°C kondensieren. Die Kondensation kann selbstverständlich auch unter erhöhtem Druck bei 105 bis 150°C durchgeführt werden. Für die Kondensationsreaktion benötigt man etwa 4 bis 12 Stunden, vorzugsweise 7 bis 9 Stunden. Pro Gewichtsteil der Arylverbindungen gemäß (a) setzt man bei der Kondensation 0,07 bis 0,17 Gewichtsteile Formaldehyd (berechnet 100 %ig) ein. Der Formaldehyd wird vorzugsweise als 10- bis 50%ige wäßrige Lösung bei der Kondensation eingesetzt. Nach Beendigung der Kondensationsreaktion wird das Reaktionsgemisch neutralisiert. Hierfür kann man Natronlauge, Kalilauge, Calciumhydroxid, Soda oder Natriumhydrogencarbonat verwenden. Der pH-Wert der Lösung, die das Kondensat enthält, wird auf Werte von 6 bis 11 eingestellt. Diese Lösung kann direkt als Dispergiermittel verwendet werden. Es ist jedoch auch möglich, daraus das Kondensationsprodukt in reiner Form oder durch Sprühtrocknung der Lösung zu gewinnen. Das Kondensationsprodukt ist leicht in Wasser löslich und eignet sich inbesondere als Dispergiermittel in Farbstoffzubereitungen. Hierfür benötigt man, bezogen auf 100 Gewichtsteile Farbstoff, 8 bis 500, vorzugsweise 25 bis 400 Gewichtsteile des Kondensationsproduktes.

Die in den Beispielen angegebenen Teile sind Gewichtsteile. Die Angaben in Prozent beziehen sich auf das Gewicht.

Für sämtliche Beispiele wurden Arylverbindungen eingesetzt, die durch fraktionierte Destillation der Spaltprodukte eines naphthenischen Rückstandsöls anfielen. Dabei wurde die bei 100 bis 120°C unter Normaldruck (1013 mbar) übergehende Fraktion verwendet. Die thermische Spaltung des naphthenischen Rückstandsöls wurde bei Temperaturen in dem Bereich von 1400 bis 1700°C durchgeführt. In der bei der fraktionierten Destillation der Spaltprodukte erhaltenen Mischungen von Arylverbindungen wurden im einzelnen folgende Stoffe identifiziert:

| Verbindung | % |
|---|---|
| Naphthalin | 44,60 |
| 2-Methylnaphthalin | 10,00 |
| 1-Methylnaphthalin | 6,20 |
| Inden | 7,40 |
| Diphenyl | 2,20 |
| Methylinden | 1,95 |
| Acenaphthen | 1,70 |
| Fluren | 1,30 |
| Indan | 1,22 |
| Phenanthren | 1,10 |
| Methylindan | 1,10 |
| Dimethylnaphthalin | 1,13 |
| Ethylnaphthalin | 0,82 |
| p-m-Xyole | 0,80 |
| Tetralin | 0,80 |
| Styrol | 0,60 |

### Beispiel 1

128 Teile des Gemisches der oben beschriebenen Arylverbindungen wurden in einem beheizbaren Reaktionsgefäß, das mit einem Rührer versehen war, vorgelegt und unter Rühren auf eine Temperatur von 70°C erwärmt. Man gab dann innerhalb von 1,5 Stunden 108 Teile Oleum, das 24 % SO₃ enthielt, zu und sorgte dafür, daß die Temperatur nicht über 75°C anstieg. Nach Zugabe des Oleums wurde das Reaktionsgemisch 2 Stunden bei 90°C und anschließend 5,5 Stunden bei 135°C gerührt. Man ließ es dann auf 70°C abkühlen, gab 150 Teile Wasser und danach 50 Teile 30 %igen wäßrigen Formaldehyd zu und kondensierte das Gemisch durch 8-stündiges Erhitzen auf einer Temperatur von 100°C. Danach fügte man 167 Teile Wasser und anschließend 51 Teile 50%ige wäßrige Natronlauge zu. Danach gab man noch 83 Teile einer 26 %igen wäßrigen Lösung von Calciumhydroxid zu, so daß sich ein pH-Wert von 4,5 einstellte. Das Reaktionsgemisch wurde filtriert und der pH-Wert des Filtrats anschließend durch Zugabe von 45 Teilen einer 18 %igen wäßrigen Natriumcarbonatlösung und 10 Teilen einer 50%igen wäßrigen Natronlauge auf 10,5 eingestellt. Die Mischung wurde dann 1 Stunde bei 90°C gerührt und mit 25 Teilen 20 %iger wäßriger Schwefelsäure versetzt, so daß sich ein pH-Wert von 8,5 einstellte. Die Lösung wurde nochmals filtriert; sie hatte einen Feststoffgehalt von 28,5 %.

In dem sulfonierten Produkt betrug das Verhältnis von α- zu β-Naphthalinsulfonsäure 1:1,4.

### Beispiel 2

Beispiel 1 wurde mit den Ausnahmen wiederholt, daß man jetzt 118 Teile Oleum mit einem SO₃-Gehalt von 24 % einsetzte und anstelle von 5,5 Stunden jetzt nur 4,5 Stunden bei 135°C sulfonierte Das Verhältnis von α- zu β-Naphthalinsulfonsäure im sulfonierten Produkt betrug 1:1,2. Die wäßrige Lösung des Dispergiermittels hatte einen Feststoffgehalt von 26 %.

### Beispiel 3

Beispiel 1 wurde mit der Ausnahme wiederholt, daß man das Reaktionsgemisch zur Sulfonierung 2 Stunden auf eine Temperatur von 140°C erhitzte. In dem sulfonierten Gemisch betrug das Verhältnis von α- zu β-Naphthalinsulfonsäure 1:1. Die im Beispiel 1 angegebene Vorschrift wurde außerdem dadurch geringfügig abgewandelt, daß man zum Reaktionsgemisch nach der Kondensation 104 Teile einer 50 %igen wäßrigen Natronlauge zusetzte und das Reaktionsgemisch auf einen pH-Wert von 11 einstellte und dann nach dem einstündigen Rühren bei 90°C den pH-Wert durch Zugabe von 21 Teilen konzentrierter Salzsäure auf einen Wert von 8,5 erniedrigte. Die so erhaltene Dispersion besaß einen Feststoffgehalt von 25 %. Das Verhältnis von α- zu β-Naphthalinsulfonsäure betrug 1:1.

### Vergleichsbeispiel 1

Beispiel 1 wurde mit der Ausnahme wiederholt, daß man die Sulfonierung mit 157 Teilen 98 %iger Schwefelsäure durchführte. In diesem Fall betrug das Verhältnis von α- zu β-Naphthalinsulfonsäure 1:5,3.

### Anwendungstechnische Beispiele

### Beispiel 4

20 Teile des blauen Dispersionsfarbstoffs der Color Index Nr. 11345 (bezogen auf das Trockengewicht) wurden in Form des wäßrigen Preßkuchens mit 15 Teilen (berechnet auf den Feststoffgehalt) des nach Beispiel 1 hergestellten Dispergiermittels, 10 Teilen Sorbit als 70 %ige wäßrige Lösung, 5 Teilen Propylenglykol, 1 Teil eines handelsüblichen Biozids (1,2-Benzisothiazolin-3-on als 9,5 %ige Lösung in Propylenglykol) und Wasser auf 100 Teile Gesamtgewicht in einem Schnellrührer angeteigt und in einer Sandmühle bis zur Erzielung einer guten Feinverteilung gemahlen. Der pH-Wert der Farbstoffdispersion betrug 8. Die Feinverteilung des Farbstoffs wurde mit Hilfe des Schleudertests (Richter und Vescia, Melliand Textilberichte 1965, 6, S. 622) und mit Hilfe des Filtertests (Schlottmann, Textilpraxis, 1957, 1, S. 63) ermittelt. Der Schleuderwert betrug: 2/3/25/70.

Die erhaltene Farbstoffzubereitung war dünnflüssig und lagerstabil und eignete sich sehr gut zum Färben von Polyesterfasern und -geweben nach allen dafür gängigen Färbeverfahren. Insbesondere beim Färben von Mischgeweben aus Polyester/Baumwolle nach dem Thermosolverfahren zeichnete sich die Farbstoffzubereitung durch eine hohe Baumwollreserve aus. Beim Färben von Wickelkörpern aus texturierten Polyesterfasern (Kreuzspulfärbung) wurden keinerlei Abfiltrationen beobachtet.

### Vergleichsbeispiel 2

Das Beispiel 4 wurde mit der Ausnahme wiederholt, daß man anstelle des dort verwendeten Dispergiermittels jetzt das gemäß Vergleichsbeispiel 1 hergestellte Dispergiermittel einsetzte. Man erhielt hierbei zwar eine lagerstabile Farbstoffzubereitung, jedoch war sie unter den Bedingungen der Wickelkörperfärbung instabil und ergab starke Ablagerungen an der Innenseite und den Stirnflächen des Garnwickels.

### Beispiel 5

17 Teile des gelben Dispersionensfarbstoffs der Color Index Nr. 47023 (bezogen auf das Trockengewicht des Farbstoffs) wurden in Form des wasserfeuchten Preßkuchens mit 12 Teilen (bezogen auf den Feststoffgehalt) des nach Beispiel 3 hergestellten Dispergiermittels, 15 Teilen Glyzerin, 1 Teil des im Beispiel 1 genannten Biozids und Wasser zur Ergänzung auf 100 Teile in einem Schnellrührer angeteigt. Der pH-Wert der Farbstoffzubereitung lag bei 8. Die Mischung wurde anschließend in einer Perlmühle bis zum Erreichen eines guten Schleuderwertes gemahlen. Der Schleuderwert betrug 6/9/22/63.

Die so erhaltene Zubereitung war dünnflüssig und lagerstabil und eignete sich hervorragend zum Färben von Geweben und Fasern aus Polyester nach allen bekannten Färbeverfahren. Beim Färben von Mischgeweben aus Polyester/Baumwolle zeigte diese Farbstoffeinstellung ein sehr geringes Anfärben des Baumwollanteils sowie eine sehr gute Auswaschbarkeit des auf der Baumwollfaser abgelagerten Dispersionsfarbstoffanteils. Auch zum Färben von Wickelkörpern aus texturierten Polyesterfasern war die Zubereitung gut geeignet.

### Beispiel 6

60 Teile (berechnet auf den Trockengehalt) des im Beispiel 1 beschrieben blauben Dispersionsfarbstoffs wurden in Form eines wasserfeuchten Preßkuchens mit 40 Teilen, bezogen auf den Feststoffgehalt, des nach Beispiel 2 hergestellten Dispergiermittels und 50 Teilen Wasser angeteigt und in einer Sandmühle vermahlen, bis eine gute Feinverteilung erreicht war. Die Feinverteilung wurde mit Hilfe des Schleuderwertes überprüft. Er betrug 4/13/25/58. Anschließend setzte man 50 Teile der 26 %igen wäßrigen Lösung des nach Beispiel 2 erhaltenen Dispergiermittels zu und erhielt auf diese Weise eine Dispersion mit einem Feststoffgehalt von 25 %. Diese Dispersion wurde in einem Zerstäubungstrockner bei einer Gaseintrittstemperatur von 120°C getrocknet.

Das dabei erhaltene Farbstoffpulver wies die in der Naßmahlstufe erreichte Feinverteilung auf. Nach Einrühren in Wasser erhielt man eine stabile Färbeflotte, die auch unter HT-Färbebedingungen keine Ausflockungen zeigte und sowohl zum Färben von Wickelkörpern aus texturierten Polyesterfasern als auch zum Thermosolfärben von Polyester/Baumwolle-Mischgewerbe sehr gut geeignet war. Die Baumwollfasern wurde hierbei nur geringfügig angefärbt. Der Dispersionsfarbstoff war von der Baumwollfaser sehr gut auswaschbar.

## Patentansprüche

1. Verfahren zur Herstellung von Kondensaten aus Arylsulfonsäuren und Formaldehyd durch Sulfonieren von Arylverbindungen und Kondensieren der Arylsulfonsäuren mit Formaldehyd in wäßrigem Medium, dadurch gekennzeichnet, daß man
(a) bei der Sulfonierung als Arylverbindungen die bei 100 bis 120°C und 1013 mbar anfallende Fraktion einsetzt, die bei der fraktionierten Destillation der Produkte der thermischen Spaltung eines naphthenischen Rückstandsöls erhalten wird,
(b) die Sulfonierung mit Oleum bei Temperaturen von 120 bis 160°C durchführt und
(c) pro Gewichtsteil der Arylverbindungen gemäß (a) 0,7 bis 1,2 Gewichtsteile Oleum, bezogen auf Oleum mit einem SO₃-Gehalt von 24 Gew.%, anwendet.

2. Verfahren nach Anspruch 1, dadurch gekennzeichnet, daß man pro Gewichtsteil der Arylverbindung gemäß (a) 0,8 bis 0,9 Gewichtsteile Oleum, bezogen auf Oleum mit einem SO₃-Gehalt von 24 Gew.%, anwendet.

3. Verfahren nach Anspruch 1 oder 2, dadurch gekennzeichnet, daß man bei der Kondensation 0,07 bis 0,17 Gewichtsteile Formaldehyd (berechnet 100 %ig), bezogen auf 1 Gewichtsteil der Arylverbindungen gemäß (a), einsetzt.

4. Verwendung der nach den Ansprüchen 1 bis 3 erhältlichen Kondensate als Dispergiermittel.

5. Verwendung nach Anspruch 4, dadurch gekennzeichnet, daß man die Kondensate als Dispergiermittel in Farbstoffzubereitungen einsetzt.

## Claims

1. A process for preparing a condensate of an arenesulfonic acid and formaldehyde by sulfonating an arene and condensing the arenesulfonic acid with formaldehyde in an aqueous medium, which comprises
(a) sulfonating an arene fraction obtained at 100-120°C and 1013 mbar in the fractional distillation of the products obtained by thermal cracking of a naphthenic residue oil,
(b) with oleum at from 120 to 160°C and
(c) using from 0.7 to 1.2 parts by weight of oleum having an SO₃ content of 24% by weight per part by weight of the arene obtained as described in (a).

2. A process as claimed in claim 1, wherein from 0.8 to 0.9 part by weight of oleum having an SO₃ content of 24% by weight is used per part by weight of arene obtained as described in (a).

3. A process as claimed in claim 1 or 2, wherein the condensation is carried out with from 0.07 to 0.17 part by weight of formaldehyde (calculated as 100% strength) per part by weight of arene obtained as described in (a).

4. The use of the condensate obtainable as claimed in claim 1 or 2 or 3, as a dispersant.

5. A use as claimed in claim 4, wherein the condensate is used as a dispersant in dye formulations.

## Revendications

1. Procédé de préparation de produits de condensation d'acides arylsulfoniques et du formaldéhyde par sulfonation de composés aryliques et condensation des acides arylsulfoniques avec le formaldéhyde en milieu aqueux, caractérisé en ce que
(a) au cours de la sulfonation, on met en oeuvre, à titre de composés aryliques, la fraction produite à une température de 100 à 120°C et une pression de 1013 mbars, que l'on obtient au cours de la distillation fractionnée des produits de la dissociation thermique d'une huile naphténique résiduaire,
(b) on entreprend la sulfonation avec de l'oléum à des températures de 120 à 160°C et
(c) on utilise, par partie pondérale des composés aryliques selon (a), 0,7 à 1,2 partie en poids d' oléum, par rapport à de l'oléum d'une teneur en SO₃ de 24% en poids.

2. Procédé suivant la revendication 1, caractérisé en ce que, par partie pondérale du composé arylique selon (a), on utilise 0,8 à 0,9 partie en poids d'oléum, par rapport à de l'oléum d'une teneur en SO₃ de 24% en poids.

3. Procédé suivant la revendication 1 ou 2, caractérisé en ce que, au cours de la condensation, on met en oeuvre 0,07 à 0,17 partie en poids de formaldéhyde (calculé à 100%), par rapport à 1 partie en poids des composés aryliques selon (a).

4. Utilisation des produits de condensation pouvant être obtenus par mise en oeuvre du procédé selon l'une quelconque des revendications 1 à 3 à titre d'agents dispersifs.

5. Utilisation suivant la revendication 4, caractérisée en ce que l'on utilise les produits de condensation à titre d'agents dispersifs dans des préparations de colorants.
